Europäisches Patentamt

European Patent Office

Office européen des brevets

(19)

(11) Publication number: **0 267 617 B1**

(12) **EUROPEAN PATENT SPECIFICATION**

(45) Date of publication of patent specification: **24.06.92**

(51) Int. Cl.5: **A61K 47/10**, A61K 47/12, A61K 47/14

(21) Application number: **87116733.4**

(22) Date of filing: **12.11.87**

The file contains technical information submitted after the application was filed and not included in this specification

(54) **Penetration enhancement with binary system of cell envelope disordering compounds and lower alcohols.**

(30) Priority: **14.11.86 US 930764**

(43) Date of publication of application:
**18.05.88 Bulletin  88/20**

(45) Publication of the grant of the patent:
**24.06.92 Bulletin  92/26**

(84) Designated Contracting States:
**AT BE CH DE ES FR GB GR IT LI LU NL SE**

(56) References cited:
**EP-A- 0 095 813**
**EP-A- 0 103 783**
**EP-A- 0 152 281**
**EP-A- 0 171 742**
**US-A- 4 537 776**

(73) Proprietor: **THERATECH, INC.**
**410 Chipeta Way**
**Salt Lake City Utah 84108(US)**

(72) Inventor: **Patel, Dinesh C.**
**5839 Meadow Crest Drive**
**Murray Utah 84107(US)**
Inventor: **Chang, Yurik**
**2689 Cavalier Drive**
**Salt Lake City Utah 84121(US)**

(74) Representative: **Patentanwälte Grünecker,**
**Kinkeldey, Stockmair & Partner**
**Maximilianstrasse 58**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to compositions which enhance the penetration of pharmaceutically-active agents through the integument. More particularly, this invention relates to binary combinations of penetration enhancers which facilitate percutaneous and transepidermal delivery of a broad range of pharmaceutically-active agents.

The resistance of the skin to being penetrated by pharmaceutically-active agents is well documented. As compared to mucosal tissues, the stratum corneum is compact and highly keratinized. The lipids and proteins of the stratum corneum, although relatively thin, is compact and quite impermeable. Such impermeability of the skin is highly essential to the well being of a living organism in that it serves as a barrier to the ingress of pathogens and toxic materials, and the egress of physiologic fluids.

The impermeability of pharmaceutical agents through the skin is due to the nature of the very thin stratum corneum layer which is only 10-15 cells, i.e. about 10 μm thick. This layer is formed naturally by cells migrating toward the skin surface from the basal layer. Cells slowly move from the basal layer to the surface where they are sloughed off. As they progress toward the surface they become progressively more dehydrated and keratinized.

Because of the advantages of dermal application of pharmaceutically-active agents, various penetration enhancers have been sought. A penetration enhancer is one or more compounds which alter the skin as a barrier to increase the flux of a desired pharmaceutical permeant across the skin.

Penetration enhancers have been primarily categorized according to their ability to enhance permeant flux via three pathways. The first is the continuous polar or aqueous pathway composed of proteins. It is thought that solvent swelling or protein conformational changes provide the key to altering the penetration of the polar pathway. Surfactants alter the transport of polar permeant molecules to a much greater extent than the transport of nonpolar permeants. Solvents such as DMSO, 2-pyrrolidone and dimethylformamide can swell the stratum corneum to also enhance the polar pathway.

The second pathway is a continuous non-polar pathway consisting of lipids. The key to altering this pathway appears to be fluidizing the lipids which, in the stratum corneum, appear to be crystalline. Solvents such as DMSO, 2-pyrrolidone, and dimethylformamide, previously mentioned also appear able to solubilize or fluidize lipids. Other solvents include diols such as glycerol and propylene glycol.

The third pathway is a heterogeneous polar-nonpolar multilaminate of lipids and proteins. Binary vehicles appear best suited to act as enhancers on this multilaminate pathway. Prior art binary systems consist of a particular category of a polar solvent combined with a variety of compounds generally referred to as "cell-envelope disordering compounds".

U. S. Patent 4,537,776, Cooper, issued August 27, 1985 contains an excellent summary of prior art and background information detailing the use of certain binary systems for permeant enhancement. That patent teaches using a binary system wherein N-(2-hydroxyethyl)pyrrolidone is used as the solvent and the cell-envelope disordering compounds are selected from methyl laurate, oleic acid, oleyl alcohol, monoolein, myristyl alcohol and mixtures thereof.

Similarly, European Patent Application 43,738, published January 13, 1982, teaches using selected diols as solvents along with a broad category of cell-envelope disordering compounds for delivery of lipophilic pharmacologically-active compounds. This reference also teaches that cosmetically acceptable solvents may also be combined with permeant and the diol and cell-envelope disordering compounds provided the solvent evaporates rapidly and completely to leave only the active components of the composition at the site of application. The acceptable solvents are stated to be ethanol or isopropanol.

Most of the cell-envelope disordering compounds mentioned in these publications are unsaturated lipid components having polar head groups.

A binary system for enhancing metoclopramide penetration is disclosed in UK Patent Application GB 2,153,223 A, published August 21, 1985 and consists of a monovalent alcohol ester of a C8-32 aliphatic monocarboxylic acid (unsaturated and/or branched if C18-32) or a C6-24 aliphatic monoalcohol (unsaturated and/or branched if C14-24) and an N-cyclic compound such as 2-pyrrolidone, N-methylpyrrolidone and the like. It is postulated that the N-cyclic compound serves as solvent function which carries the active agent whereas the esters or alcohols serve as adjuvants to open up the stratum corneum, i.e. as cell-envelope disordering compounds.

In referring to the epidermal permeability of lower alkanols, Drug Delivery Systems, Characteristics and Biomedical Applications, Oxford University Press, NY, 1981, edited by R. L. Juliano, teaches at page 159 that simple alcohols through n-butanol have epidermal permeabilities no different than that of water. However, Campbell et al, U.S. Patent 4,379,454; Campbell et al, U.S. Patent 4,460,372 and Gale et al, U.S. Patent 4,588,580 refer to the use of gelled ethanol as an enhancer in specialized transdermal or percutaneous drug delivery devices.

From the above cited art and incorporated disclosures, it is apparent that some binary enhancers favor lipophilic permeants. There appears to be no recognition of an enhancer system that favors the penetration of salts and other hydrophilic permeants. Moreover, those binary systems containing diols and N-cyclic solvents may cause considerable skin irritation even at low concentrations. However, diols or N-cyclic solvents are taught to be necessary components of a binary system. In general, simple alcohols are taught to be cosolvents to help bring various mixtures into solution but are to be evaporated rapidly from the skin surface and do not function as penetration enhancers any better than water.

Other patents or publications relating to transdermal administration of active permeants are Cooper, European Patent Application 95,813,A2, published July 12, 1983, entitled Penetrating Topical Pharmaceutical Compositions Containing 9-(2-Hydroxyethoxymethyl)Guanine; Durrant et al, European Patent Application 117,080, published August 29, 1984 entitled Skin Treatment Composition.

An object of the present invention is to provide compositions which allow improved penetration of a broad category of pharmaceutically-active agents which are lipophilic or hydrophilic including salts and which further produce little or no skin irritation to human or animal tissue systems.

This object is achieved by a penetration-enhancing pharmaceutical composition for topical application comprising:

(a) 0.01 to 50% by weight of an active pharmaceutical permeant contained in,

(b) 40-99.99% by weight of a binary penetration-enhancing vehicle exclusive of diols and N-cyclic solvents comprising

(i) one or more cell-envelope disordering compounds selected from oleic acid, glycerol monooleate, glycerol dioleate and glycerol trioleate and mixtures thereof, and

(ii) a lower alkanol selected from ethanol, propanol and isopropanol and mixtures thereof, wherein the weight ratio of cell-envelope disordering compound to lower alkanol is between 50:1 and 1:50; and

(c) optionally, such inert components as are needed to improve the cosmetic acceptability of the composition and which do not interfere with the penetration enhancement properties of the binary penetration-enhancing vehicle.

By employing this binary penetration-enhancing vehicle, it has been found that significant penetration of salts and other hydrophilic permeants as well as lipophilic permeants is obtained and that skin irritation often associated with cell-envelope disordering compounds and/or solvents is essentially nonexistent.

The invention is therefore not limited to any specific category or categories of permeants but is inclusive of all therapeutically active compounds and their use which are responsive by being incorporated into the binary penetration-enhancing vehicle as more fully set forth herein.

The following definitions, when used and as they apply to the present invention, are consistent with those contained in U. S. Patent 4,537,776.

By "topical administration" or "topical application" is meant directly laying or spreading upon epidermal tissue, especially outer skin or membrane, including the skin or membrane of the oral or vaginal cavities.

By "safe and effective", is meant a sufficient amount of the permeant composition to provide the desired systemic effect and performance, or local activity, or both at a reasonable benefit/risk ratio attendant any medical treatment. Within the scope of sound medical judgment, the amount of permeant used will vary with the particular condition being treated, the severity of the condition, the duration of the treatment, the specific permeant compound employed, its concentration, the condition of the patient, concurrent therapies being administered and other factors within the knowledge and expertise of the patient or the attending physician or other practitioner.

By "toxicologically- or pharmaceutically-acceptable" is meant the pharmaceutical actives (or permeants), as well as the other compatible drugs, medication or inert ingredients which the term describes, are suitable for use in contact with the tissues of humans and animals without undue e.g., toxicity, irritation or allergic response, commensurate with a reasonable benefit/risk ratio.

By the term "comprising" is meant that various other compatible drugs and medicaments, as well as inert ingredients, occlusive agents, and cosmetic vehicles, can be conjointly employed in the compositions of this invention, as long as the critical binary penetration enhancement vehicle and pharmaceutically active permeant are used.

By "afflicted situs" is meant a localized area of pathology, discomfort, infection, inflammation or lesion, and the immediately surrounding area.

By "application situs" is meant a site suitable for topical application with or without the means of a mechanical sustained release device, patch or dressing, e.g. behind the ear, on the arm, back, chest, stomach, leg, top of foot.

By "penetration-enhancing" is meant that the binary penetration enhancing carriers or vehicles of this

3

invention provide marked transepidermal or percutaneous delivery of an incorporated active permeant, when compared to other compositions at equal chemical potential. Equal chemical potential is important since varying solubilities of drugs in different carrier vehicles will affect their transport across skin. As stated in U. S. Patent 4,537,776, if a drug is soluble in vehicle A to the extent of 24%, and in vehicle B to the extent of 4%, were the compositions to be compared at equal percentage concentration, rather than equal chemical potential, the lower solubility vehicle would show a misleading six-fold difference in transport over the more soluble vehicle. Therefore, the simplest way of assuring equal chemical potential for evaluating penetration enhancement is to use saturated solutions or solutions of equal percentage of saturation of active permeants in the various enhancer combinations, e.g. 50% saturated. In the examples used herein, the enhancer combinations are saturated with the active permeant components.

As used herein, all percentages and ratios are by weight of the total composition unless otherwise specified.

The terms "permeant", "active", "pharmaceutical active", "pharmacological active", "pharmaceutical agent", "pharmacological agent", "pharmaceutically-, or pharmacologically-active agent", "chemical agent", "therapeutic agent", and "drug", are used interchangeably herein.

The compositions of this invention require, at a minimum, a permeant capable of producing systemic effects, or producing or possessing local activity, in a binary vehicle or carrier comprising a cell-envelope disordering compound and a lower alcohol selected from ethyl alcohol, propyl alcohol or isopropyl alcohol.

The composition may also contain other optional components which enhance their cosmetic appeal or acceptability, i.e., for example, thickeners, pigments, fragrances and perfumes. The binary penetration combinations are essentially free of skin irritation characteristics. However, a permeant combined with the penetration enhancers may cause some irritation. Therefore, if desired other components which tend to reduce skin irritation may be incorporated into the compositions.

The binary penetration enhancement combinations of the present invention significantly enhance the penetration of a host of pharmaceutically-active permeants including salts. These permeants may be lipophilic or hydrophilic or partially lipophilic or hydrophilic.

The binary combinations comprise one or more cell-envelope disordering compound and a lower alkanol selected from the group consisting of ethanol, propanol and isopropanol.

The cell envelope disordering compounds are known in the art as being useful in topical pharmaceutical preparations. These compounds are thought to assist in penetration by disrupting or disordering the lipid structure of the stratum corneum cell-envelopes.

Numerous specific cell envelope disordering compounds are listed in European Patent Application 43,738. The cell envelope disordering compounds for use in combining with ethanol, propanol or isopropanol to form penetration enhancing compositions of the present invention are selected from oleic acid, glycerol trioleate, glycerol dioleate, glycerol monooleate (monoolein) and mixtures thereof. Particularly preferred are oleic acid and glycerol dioleate and mixtures thereof.

Binary mixtures of a $C_2$-$C_3$ alcohol and any of the above referenced cell-envelope disordering compounds, in a weight:weight ratio of alcohol to cell-envelope disordering compound of 50:1 to 1:50, provide significantly enhanced penetration for the permeants described herein. A weight:weight ratio of alcohol:cell-envelope disordering compounds of from 1:9 to 9:1 is preferred.

The compositions of the invention typically contain from 40 to 99.99%, and preferably 70 to 99.9%, by weight of the overall composition, of the penetration enhancing binary mixture of $C_2$ or $C_3$ alcohol and the cell-envelope disordering compound employing the ratios described above. The exact percentages may be readily determined by one having ordinary skill in the art. All that is required is that an effective amount of the active permeant be incorporated into the penetration enhancing composition with or without being combined with other ingredients.

The binary penetration enhancers of the present invention may be formulated to incorporate a broad range of pharmaceutically-active permeants. One of the distinct advantages of these enhancer combinations is that they function to enhance penetration of both lipophilic and hydrophilic permeants including salts and are virtually free from skin irritation effects. The compositions of this invention may be utilized in delivering active permeants to the "target" areas as mentioned in U. S. Patent 4,537,776, i.e. (1) at the surface of the skin; (2) in the stratum corneum itself; (3) in the viable epidermis and upper dermis, just below the stratum corneum; (4) in the various glands and structures in and beneath the dermis (e.g., subcutaneous adipose, dermal vasculature); and/or (5) the general system (i.e. systemic effects).

In view of this, the invention is not limited to any specific type or class of active permeants. Based on the parameters contained herein it is within the ability of one having ordinary skill in the art to determine which permeants can be utilized. Some routine experimentation or testing may be required to determine optimum conditions such as exact concentrations of permeants and ratios of cell-envelope disordering compounds to alcohols. Also, some permeants may work best with one particular class of cell-envelope disordering compounds and/or alcohols. The screening of all possible combinations and ratios of permeants, cell-envelope disordering compounds and $C_2$ and $C_3$ alcohols has not been attempted.

However, based on the formulation of a representative sampling of diverse active permeants, it is apparent that the binary combination of a cell-envelope disordering compound and a $C_2$ or $C_3$ alcohol will function to enhance the penetration of a broad spectrum of pharmaceutically-active permeants. Such agents include, those mentioned in U. S. Patent 4,537,776 such as antimicrobials, antibacterials, antibiotics, antimyobacterials, antimalarials, antiamebics, anthelmintics, antifungals, antivirals, neoplastic agents, agents affecting the immune response, blood calcium regulators, peptide and protein hormones, male sex hormones, female sex hormones, agents useful in glucose regulation, anticoagulents, antithrombotics and hemostatics, antihyperlipidemic agents, cardiac drugs, thyromimetic and antithyroid drugs, adrenergics, antihypertensive agents, cholinergics, anticholinergics, antispasmodics, antiulcer agents, skeletal and smooth muscle relaxants, histamine $H_2$-receptor agonists and antagonists, prostaglandins, general inhibitors of the allergic response, antihistamines, local anesthetics, analgesics, antitussives, sedative-hypnotic agents, anticonvulsants, antipsychotics, anti-anxiety agents, antidepressant agents, anorexigenics, non-steroidal anti-inflammatory agents, steroidal anti-inflammatory agents, bone-active agents, antiarthritics, vitamins, diagnostic agents and sunscreens. These agents can be used for systemic effect, local activity, or both, as appropriate. Examples of pharmaceutically-active permeants are well-known in the art and can be found listed in sources identified in U. S. Patent 4,537,776 as well as others. For example, active agents, in approved commercially available formulations, their recommended dosages, adverse reaction and side effects are listed in the annual publication of the Physicians' Desk Reference, published by Medical Economics Company, a division of Litton Industries, Inc.

The pharmaceutically-active permeants may be used in the compositions and methods of the present invention at any safe and effective level, or in any safe and effective amount. Dosages will obviously be a function of various variables, such as how active the agent is, how soluble it is in the penetration enhancing composition, how often it is to be applied, whether the use is to be topical (applied to the "afflicted situs") or systemic (applied to the "application situs"), whether two or more active permeants are to be combined and the particular patient being treated.

In any event the dosage will be the smallest that will achieve the desired result and the period of administration will be as short as possible to attain this result.

In general, dosages and means of application as taught in U. S. Patent 4,337,776 are appropriate to the present invention. Levels of active permeants may vary from 0.01% to 50% by weight of the total composition with levels of from 0.01 to 30% being preferred. Levels from 0.05 to 15% being especially preferred and levels of from 0.1 to 10% being most especially preferred for some active permeants. However, for some active permeants it may be required to use more or less than stated above to attain the desired results. Hence, the invention is not directed to any particular amount of active ingredient as long as it is safe and effective.

A compendium of active permeants is contained in U. S. Patent 4,537,776 and published European Patent Application 43,738. However, for purposes of illustration a more concise listing of active agents follows.

Typical antihypertensive agents which may be utilized include, minoxidil, nadolol, pargyline, pindolol, propranolol, reserpine, timolol, trimethaphan, metoprolol, hydrochlorothiazide, hydralazine, furosemide, clonidine and chlorthalidone.

Diuretics include, benzthiazide, buthiazide, cyclopenthiazide, cyclothiazide, metolazone, triamterelene, chlorazamil, clazolimme, and hydroflumethiazide.

Exemplary of anorexigenics are, amphetamine, methamphetamine, chlorphentermine, chlortermine, phentermine, phendimetrazine, mazindol, oxazoline, and phenoxyalbyleneamine.

Fungistatic and fungicidal agents encompass, thiabendazole, chloroxine, fungimycin, griseofulvin, chlordantoin, salicylic acid, nystatin, clotrimazole, fezatione, socium pyrithione, amphotericin B, 5-fluorocytosine, haloprogin, vifampin, and pimaricin.

A broad range of analgesics may be utilized including, morphine, codeine, heroine, methadone, thebaine, orpiarine, buprenorphine, morphinans, benzomorphans, acetaminophen, butorphanol, diflunisal, fenoprofen, fentanyl, fentanyl citrate, hydrocodone, ibuprofen, oxymorphone, pentaxicine, naproxen, nalbuphine, mefenamic acid, meperidine and dihydroergotamine.

Exemplary antitussive agents include, diphenhydramine, guaifenesine, hydromorphone, ephedrine, phenylpropanolamine, theophylline, codeine, noscapine, levopropoxyphene, carbetapentane, chlorpehndianol and benzonatate.

Among the sedatives which may be utilized are, chloralhydrate, butabarbital, alprazolam, amobarbital, chlordiazepoxide, diazepam, mephobarbital, secobarbital, diphenhydramine, ethinamate, flurazepam, halazepam, haloperidol, prochlorperazine, oxazepam, and talbutal.

Examples of cardiac drugs are, quinidine, propranolol, nifedipine, procaine, dobutamine, digitoxin, phenytoin, sodium nitroprusside, nitroglycerin, verapamil HC1, digoxin, nicardipine HC1, and isosorbide dinitrate.

Antimicrobial agents are inclusive of, erythromycin, suflonamide, lincomycin, clindamycin, tetracycline, chlortetracycline, demeclocycline, doxycycline, and methacycline.

Examples of useful antibacterial agents are, phenols, hydroxy benzoic acid, hydroxy quinoline, nitrofuran, nitroimidazoles, oxolinic acid, actinomycetin, bacitracin, tyrothricin, kanamycin, neomycin and chloramphenicol.

Steroidal anti-inflammatory agents are illustrated by, triamcinolone acetonide, beclomethasone dipropionate, hydrocortisone acetate, fluocinolone acetonide, betamethasone valerate, prednisolone, prednisone, methyl prednisolone and paramothasone.

Inclusive of non-steroidal anti-inflammatory agents are acetyl salicylic acid, fenoprofen calcium, ibuprofen, indomethacin, meclofenamate sodium, mefenamic acid, naproxen sodium, phenylbutazone, and oxyphenbutazone.

Anti-emetics ire illustrated by, thiethylperazine, metoclopramide, cyclizine, meclizine, prochlorperazine, doxylamine succinate, promethazine, triflupromazine, and hydroxyzine.

Exemplary amino acid, peptide and protein hormones include, thyroxine, growth hormone (GH), interstital cell stimulating hormone (ICSH), follicile-stimulating hormone (FSH), thyrotropic hormone (TSH), andrenocorticotropic hormone (ACTH), vasopressin and their active degradation products. Some products may have sufficiently high molecular weights that absorption through the stratum corneum or mucous membranes may be difficult. Therefore, the invention is applicable only to those hormones which have molecular weights and stereoconfigurations which will allow passage through the skin.

Female sex hormones which can be used include, estradiol, diethylstilbestrol, conjugated estrogens, estrone, northindrone, medroxyprogesterone, progesterone, and norgestrel.

Typical male sex hormones which may be utilized may be represented by, testosterone, methyltestosterone, and fluoxymesterone.

The above listed active permeants may, along with others not specifically disclosed, be used separately or in combination according to the treatment regimen desired.

Preferred categories of active permeants include anti-hypertensive agents, cardiac drugs, analgesics, sedative-hypnotic agents, anti-anxiety agents, steriodal anti-inflammatory agents, non-steroidal anti-inflammatory agents, male sex hormones, and female sex hormones. Those active permeants specifically listed above under each category are particularly preferred.

The components of this invention are inclusive of the active permeants combined with the binary penetration enhancing mixture of cell-envelope disordering compounds and C2 and C3 alcohols. It is contemplated that compositions containing only these ingredients will be sufficient in most instances to obtain the desired results. However, in preparing formulations for actual use, it may be desirable to add other components such as excipients, dyes, perfumes, fragrances, opacifiers, thickening agents, preservatives, anti-oxidants, gelling agents, surfactants and stabilizers. For example, when forming gels or cremes, it may be desirable to add significant amounts of water, i.e. up to 50% in some cases for gels. Such materials, when added, should not unduly interfere with the penetration enhancement of these compositions. Such formula modifications to improve cosmetic acceptability are well within the skill of workers in the art and do not form part of the present invention.

In any form of medical practice, there are many variables which affect the particular treatment regimen. In that regard, the final diagnosis and treatment is left to the expertise of the practitioner and patient. As previously stated, in clinical practice, it is the goal that the dosage of any active permeant be as small as possible to achieve the result desired and that the duration of the administration of the permeant be as short as possible. To attain these conditions, it is imperative that the amount of active ingredient utilized is a safe and effective amount whether applied to an afflicted situs or an application situs. When local treatment is desired, the compositions are applied to the afflicted situs. When systemic treatment is desired, the compositions are applied to an application situs, preferably from a sustained release device such as a patch, bondage, web, film or the like. When both local and systemic treatments are indicated, the compositions can be applied at both the afflicted situs and application situs, or both. The selection of active permeant or combination of permeants and particular penetration enhancement combination are necessarily left to the skill of the practitioner provided the parameters outlined herein are followed.

The dosage, rate of application, place of application, and other treatment parameters are generally outlined in U.S. Patent 4,537,776. What is a safe and effective amount of any ingredient will obviously depend upon the active ingredient being used, the site of application, the effectiveness of the penetration enhancer and other parameters outlined herein.

A practitioner being skilled in the art will be able to determine the application parameters of each specific formulation based on the needs of each patient.

The following examples demonstrate the penetration enhancement which is obtained by the binary cell envelope disordering compounds-lower alkanol compositions. In making these tests human skin consisting of heat-separated abdominal epidermis, taken at autopsy, was placed in a standard Franz diffusion apparatus in a horizontal position between a lower, capped diffusion cell and an upper open cell. A normal saline solution was added to the lower diffusion cell in contact with the subcutaneous side of the skin, and the test composition, consisting of a saturated solution of an active drug being monitored formulated in the binary penetration enhancer, was added to the diffusion cell in contact with the upper or epidermal side of the skin.

The cell assembly was kept in a constant-temperature room at about 37 degrees C. At predetermined intervals, the diffusate from the cell on the subcutaneous side of the skin was withdrawn and the amount of drug in the diffusate was measured using standard analytical techniques. Each test was run on a separate skin sample. In each case the amount of active drug used was that required to form a saturated solution. The results are reported in terms of flux, $[\mu g(mcg)/cm^2/day]$ or relative flux.

EXAMPLE I

To show the penetration enhancement effects of the binary cell envelope disordering compound-lower alkanol compositions are applicable to active agents inclusive of hydrophilic, salts and hydrophobic agents the following compositions were tested.

| Flux $[\mu g(mcg)/cm^2/day]$ | | | |
|---|---|---|---|
| Test No. | Active Ingredient | Enhancement System | Flux |
| I-A | Estradiol | Propylene Glycol<br>Glycerol Dioleate /(GDO)<br>GDO/Ethanol (80:20 w/w) | 14.9<br>14.3<br>20.9 |
| I-B | Na-Salicylate | Propylene Glycol<br>Glycerol Dioleate (GDO)<br>GDO/Ethanol (80:20 w/w) | 138.6<br>6,626.0<br>13,694.4 |
| I-C | Ara-A | Propylene Glycol<br>Glycerol Dioleate (GDO)<br>GDO/Ethanol (80:20 w/w) | 0.44<br>0.48<br>3.98 |

The combination of ethanol and GDO shows substantial penetration enhancement effects as compared to a diol (propylene glycol) or GPO alone for all three active agents.

EXAMPLE II

A series of tests similar to Example I were conducted utilizing a greater variety of active agents with various component combinations forming penetration enhancement systems which were directly compared with individual components making up the systems as follows:

| FLUX [$\mu$g(mcg)/cm$^2$/day] | | | | | | |
|---|---|---|---|---|---|---|
| Test No. | Enhancement System (%w/w) | Estradiol | Prednisolone | Propranolol HCL | Minoxidil HCL | Sodium Salicylate |
| II-A | 40% OA<br>40% GDO<br>20% EtOH | 75.6 | 915.7 | 1,216.0 | 383.6 | 12,838.0 |
| II-B | 80% OA<br>20% EtOH | 21.2 | 462.0 | 889.0 | 303.0 | 22,905.0 |
| II-C | 80% GDO<br>20% EtOH | 48.6 | 571.0 | 767.0 | 197.0 | 12,541.0 |
| II-D | 95% PG<br>5% OA | 120.1 | 164.0 | 2,259.0 | 1,155.0 | 834.0 |
| II-E | 100% OA | 31.8 | 291.0 | 258.0 | 221.5 | 18,349.0 |
| II-F | 100% EtOH | 18.7 | 81.0 | 45.0 | 23.4 | 1,094.0 |
| II-G | 100% PG | 2.6 | 5.0 | 25.0 | 14.6 | 231.8 |
| II-H | 100% GDO | 12.1 | 92.0 | 257.0 | 62.1 | - |
| Note: EtOH = Ethanol<br>PG = Propylene Glycol<br>GDO = Glycerol Dioleate<br>OA = Oleic Acid | | | | | | |

The penetration enhancer composition utilized in Test II-D is taught in the prior art and shown in Example XIV of European Patent Application 43,738 and generally provides for excellent skin penetration enhancement. However, as shown in following Example VI, this combination of oleic acid and propylene glycol causes severe skin irritation. The penetration enhancement systems of Tests II-A, II-B and II-C, with minor exceptions, showed across the board improvement in penetration enhancement over the individual components used alone and generally greater than additive enhancement effects which one would expect when combining these ingredients.

EXAMPLE III

Following the procedure and penetration enhancement systems of Example II, the relative flux of haloperidol as active agent was determined. The results are as follows:

| Test No. | Enhancement System | Relative Flux Haloperidol |
|---|---|---|
| III-A | 40% OA<br>40% GDO<br>20% EtOH | 19.1 |
| III-B | 80% OA<br>20% EtOH | 10.8 |
| III-C | 80% GDO<br>20% EtOH | 21.7 |
| III-D | 95% PG<br>5% OA | 22.4 |
| III-E | 100% OA | 5.2 |
| III-F | 100% EtOH | 5.1 |
| III-G | 100% PG | 1.0 |
| III-H | 100% GDO | 12.2 |

Note: EtOH = Ethanol
PG = Propylene Glycol
GDO = Glycerol Dioleate
OA = Oleic Acid

It is evident from the above that the combinations of glycerol dioleate and/or oleic acid with ethanol provide penetration enhancement similar to that obtained with propylene glycol and oleic acid and, as will subsequently be demonstrated, does not possess the skin irritation properties of propylene glycol-oleic acid combinations. The enhancement obtained by combining GDO and oleic acid cell-envelope disordering agents with ethanol was far greater than that obtained utilizing the individual components alone.

EXAMPLE IV

To show the efficacy of other cell-envelope disordering compounds in combination with lower alkanols as penetration enhancers, a series of tests were run with propranolol as the active agent. The procedure as outlined in the above examples was followed to determine flux. All enhancer combinations consisted of 75% weight of a cell-envelope disordering compound and 25% weight isopropyl alcohol (i-PrOH). The results are as follows:

9

| Test No. | Enhancement System (75% w/25% w) | Flux $[\mu g \ (mcg)/cm^2/day]$ |
|---|---|---|
| **Esters** | | |
| IV-A | GDO + i-PrOH | 1,814.0 |
| IV-B | Methyl Laurate + i-PrOH | 5,534.0 |
| IV-C | Lauryl Oleate + i-PrOH | 571.0 |
| IV-D | Methyl Vaccenate + i-PrOH | 406.0 |
| IV-E | Cis-5-Decenyl Acetate + i-PrOH | 5,534.0 |
| **Higher Alcohols** | | |
| IV-F | Oleyl Alcohol + i-PrOH | 4,553.0 |
| IV-G | Hexadecenol + i-PrOH | 7,055.0 |
| IV-H | Dodecanol + i-PrOH | 5,451.0 |
| IV-I | Vacenyl Alcohol + i-PrOH | 6,481.0 |
| IV-J | Decanol + i-PrOH | 7,102.0 |
| IV-K | Octanol + i-PrOH | 6,852.0 |
| **Fatty Acids** | | |
| IV-L | Oleic Acid + i PrOH | 860.0 |
| IV-M | Petroselenic Acid + i-PrOH | 856.0 |
| IV-N | Linoleic Acid + i-PrOH | 2,358.0 |
| IV-O | Linolelaidic Acid + i-PrOH | 1,384.0 |
| IV-P | Linolenic Acid + i-PrOH | 1,204.0 |
| IV-Q | Vaccenic Acid + i-PrOH | 673.0 |

While there is no direct comparison utilizing the cell- envelope disordering components of the enhancement combination alone, it has been previously shown that the combinations of enhancers as shown in Tests IV-A and IV-L, utilizing ethyl alcohol instead of isopropyl alcohol, show marked penetration enhancement of active ingredients. Using Tests IV-A and IV-L as the standards, the other cell envelope disordering components, with few exceptions, show comparable or superior penetration enhancement of propranolol.

EXAMPLE V

Again, following the procedure of the preceding examples, a series of tests utilizing glycerol dioleate and/or oleic acid as cell-envelope disordering components combined with isopropyl alcohol as penetration enhancers, were performed using propranolol HCl and testosterone as active agents. Results are as follows:

| Test No. | Enhancement System (% w/w) | Active Agent | Flux $[\mu g (mcg)/cm^2/day]$ |
|---|---|---|---|
| **Skin Sample #1** | | | |
| V-A | 100% GDO | Propranolol HCl | 448.8 |
| V-B | 100% i-PrOH | Propranolol HCl | 67.2 |
| V-C | 80% GDO 20% i-PrOH | Propranolol HCl | 1,008.2 |
| **Skin Sample #2** | | | |
| V-D | 100% GDO | Testosterone | 120.0 |
| V-E | 100% OA | Testosterone | 144.0 |
| V-F | 80% OA 20% i-PrOH | Testosterone | 456.0 |
| V-G | 80% OA 10% i-PrOH 10% GDO | Testosterone | 912.0 |
| **Skin Sample #3** | | | |
| V-H | 100% OA | Testosterone | 76.8 |
| V-I | 80% OA 20% i-PrOH | Testosterone | 374.4 |
| V-J | 80% GDO 20% i-PrOH | Testosterone | 384.0 |

i-PrOH = Isopropanol

GDO = Glycerol Dioleate

OA = Oleic Acid

The above show that enhancers consisting of both cell-envelope disordering compounds and isopropanol are clearly superior to the individual components used separately.

EXAMPLE VI

The following test was conducted in order to show that skin penetration enhancers, used in the above examples, to facilitate the passage of medicinal compounds through the skin and consisting of combinations of cell-envelope disordering agents combined with a lower alkanol, produce less skin irritation and sensitization than prior art penetration agents consisting of a cell-envelope disordering compound and a diol.

Twenty four healthy adults (sixteen females and eight males) between the ages of 18-65 were selected for the test. Each subject was selected without regard to race or sex. However, each subject was required to be in good health and meet required criteria regarding allergies, skin cancer, medications, recent surgery, etc.

Eight different enhancer compositions (Test Substances I through VIII) were prepared as follows (ingredients reported in % w/w):

I. Propylene glycol:Oleic Acid (95:5).

II. Glycerol dioleate:Ethanol (80:20).

III. Glycerol dioleate:Ethanol (20:80).

VI. Glycerol dioleate:Glycerol formal:Ethanol (70:10:20).

VIII. Glycerol dioleate:Oleic acid:Ethanol (70:10:20).

Test solutions were prepared and stored at room temperature until used. When ready for use 150 µl of

11

test solution was placed on an adhesive bandage patch consisting of a 4,8 cm$^2$ (3/4 inch square) nonwoven cotton pad, Webril (TM) (Kendall Corporation) which retained the test substance, backed by a 9,7 cm$^2$ (1.5 inch square) of blenderm type tape (3M Company) to hold the pad onto the surface of the skin. The patches were individually packaged with a peeloff backing.

The backing was peeled off and the test solution applied to the patch. The test area was cleaned with a gauze pad saturated with 95% ethyl alcohol. The patches were then applied to the lateral surface of one upper arm of the subject in a designated sequence to eliminate position and order grading bias.

The best subjects were divided into two application groups (Group A and Group B) of twelve subjects each. Each group was further subdivided into four groups each containing three subjects. Each subject in Group A was treated with test Substances I, II, III and IV and each subject in Group B was treated with test Substances V, VI, VII and VIII. The positioning of substances was the same in each subgroup, but the positioning varied from one subgroup to another according to a randomization code.

The test consisted of nine 24 hour patch exposure (induction period) to the same test site of each subject with a 24 to 48 hour rest period between each exposure. About two weeks after the last induction patch was applied, the original test site and an alternate test site were challenged with a 24 hour patch exposure to the test material.

Each subject was instructed to keep all patches as dry as possible and to remove and discard them after approximately 24 hours. The patch area could be cleaned in a normal manner after removal but the subjects were cautioned not to swim when the patches were in place. There were no bathing or showering restrictions.

Induction patches were applied on Monday, Wednesday and Friday for the first three weeks of the test. The test sites were scored using an artificial light source to illuminate the patch area prior to the application of each new patch during the induction tests with a final reading being taken on the Monday following the ninth application Two weeks after the final reading of the induction tests, challenge patches were applied simultaneously to both the original (at the original sites) and opposite arms (in a similar position) of each subject. These patches were worn for 24 hours and then discarded. The challenge test sites were graded on the second and fourth days following their application.

Test sites were scored as follows: O = no visible reaction; 1 = mild reaction, erythema; 1E = mild erythematous reaction with papules and/or edema; 2 = moderate reaction, erythema; 2E = moderate erythematous reaction with papules and/or edema; 3 = strong reaction, erythema; 3E = strong erythematous reaction with marked edema, papules and/or few vesicles, 4 = severe reaction with erythema, edema, papules and vesicles (may be evidence of weeping); 5 = bullous reaction; [S] = reaction spread beyond patch area; and N9G = No 9th induction grade. Gradings containing two numbers, e.g. 1/1E, are readings from two application sites when skin irritation at the initial test site required the moving of test patches from one test site to another.

The patch site grades are as follows:

EP 0 267 617 B1

Test Substance - I

| Subject Drug Number | \- Induction Grades - | | | | | | | | | Challenge Grades | | | |
|---|---|---|---|---|---|---|---|---|---|---|---|---|---|
| | | | | | | | | | | Orig 1 | Alt 1 | Orig 2 | Alt 2 |
| | 1 | 2 | 3 | 4 | 5 | 6 | 7 | 8 | 9 | | | | |
| 01 | 1 | 0 | 1 | 2 | 1 | 1 | 1E | 1/1 | 1/1E | 1 | 1 | 1 | 1 |
| 02 | 1 | 1 | 0 | 1 | 1 | 1E | 2E | 1E/0 | 1/3E | 2E | 2E | 3E | 3E |
| 03 | 1 | 1 | 1E | 1E(S) | 1/1E | 3E(S)/2E(S) | 2E/1 | 1E/1 | 1/1 | 5(S) | 4(S) | 3E | 2(S) |
| 04 | 0 | 1 | 1 | 1 | 1E(S) | 1/1E | 1E/1 | 2E/1 | 1/1 | 3E | 2E | 2E | 2 |
| 05 | 1E | 1 | 2E | 1E/3E(S) | 2E/1E | 1/4 | 1/2E | 1/1 | 1/1E | 1 | 0 | 1 | 0 |
| 06 | 1 | 1 | 1E | 2E(S) | 2/2E | 3E/2E | 3E/3 | 2/2 | N9G | * | * | * | * |
| 07 | 1 | 1 | 1 | 1 | 1 | 1E | 2 | 1/1E | 1E/2E | 2E | 2E | 1E | 1E |
| 08 | 1 | 1 | 1 | 1 | 1 | 1 | 1E | 1/1E | 1/2E | 2E | 3 | 1E | 2E |
| 09 | 0 | 0 | 1 | 1 | 1 | 1E | 2E | 1E/1E | 1/2E | 3E | 3E | 3E | 3E |
| 10 | 1 | 1 | 1 | 1 | 1 | 1 | 2E | 0/1E | 1/3E | 5(S) | 4 | 4 | 2E |
| 11 | 1 | 1 | 3 | 1 | 1E | 2E | 2/5 | 1/3E | 1/1E | 2E | 2E | 1E | 1E |
| 12 | 1E | 1 | 2 | 3 | 2 | 1/2E | 2/1E | 1E/1E | 1/1 | 2E | 3E | 1E | 2E |

* Patch not applied.

Test Substance - 11

| Subject Drug Number | Induction Grades | | | | | | | | | Challenge Grades | | | |
|---|---|---|---|---|---|---|---|---|---|---|---|---|---|
| | 1 | 2 | 3 | 4 | 5 | 6 | 7 | 8 | 9 | Orig 1 | Alt 1 | Orig 2 | Alt 2 |
| 01 | 0 | 0 | 0 | 0 | 0 | 0 | 0 | 0 | 0 | 0 | 0 | 0 | 0 |
| 02 | 0 | 0 | 0 | 0 | 0 | 0 | 0 | 0 | 0 | 0 | 0 | 0 | 0 |
| 03 | 0 | 0 | 0 | 0 | 0 | 0 | 0 | 0 | 0 | 0 | 0 | 0 | 0 |
| 04 | 0 | 0 | 0 | 0 | 0 | 0 | 0 | 0 | 0 | 0 | 0 | 0 | 0 |
| 05 | 0 | 0 | 0 | 0 | 0 | 0 | 0 | 0 | 0 | * | * | * | * |
| 06 | 0 | 0 | 0 | 0 | 1 | 1 | 1 | 1 | N9G | * | * | * | * |
| 07 | 0 | 0 | 0 | 0 | 0 | 0 | 0 | 0 | 0 | 0 | 0 | 0 | 0 |
| 08 | 0 | 0 | 0 | 0 | 0 | 0 | 0 | 0 | 0 | 0 | 0 | 0 | 0 |
| 09 | 0 | 0 | 0 | 0 | 0 | 0 | 0 | 0 | 0 | 0 | 0 | 0 | 0 |
| 10 | 0 | 0 | 0 | 0 | 0 | 0 | 0 | 0 | 0 | 0 | 0 | 0 | 0 |
| 11 | 0 | 0 | 0 | 0 | 0 | 0 | 0 | 0 | 0 | 0 | 0 | 0 | 0 |
| 12 | 0 | 0 | 0 | 0 | 0 | 0 | 0 | 0 | 0 | 0 | 0 | 0 | 0 |

* Patch not applied.

EP 0 267 617 B1

Test Substance - III

| Subject Drug Number | Induction Grades | | | | | | | | | Challenge Grades | | | |
| --- | --- | --- | --- | --- | --- | --- | --- | --- | --- | --- | --- | --- | --- |
| | | | | | | | | | | Orig 1 | Alt 1 | Orig 2 | Alt 2 |
| | 1 | 2 | 3 | 4 | 5 | 6 | 7 | 8 | 9 | | | | |
| 01 | 0 | 0 | 0 | 0 | 0 | 0 | 0 | 0 | 0 | 0 | 0 | 0 | 0 |
| 02 | 0 | 0 | 0 | 0 | 0 | 0 | 1E | 1 | 1 | 0 | 0 | 0 | 0 |
| 03 | 0 | 0 | 0 | 0 | 0 | 0 | 0 | 0 | 0 | 0 | 0 | 0 | 0 |
| 04 | 0 | 0 | 0 | 0 | 0 | 0 | 0 | 0 | 0 | 0 | 0 | 0 | 0 |
| 05 | 0 | 0 | 0 | 0 | 0 | 0 | 0 | 0 | 0 | 0 | * | 0 | * |
| 06 | 0 | 0 | 0 | 0 | 1 | 1 | 1 | 0 | N9G | * | * | * | * |
| 07 | 0 | 0 | 0 | 0 | 0 | 0 | 0 | 0 | 0 | 0 | 0 | 0 | 0 |
| 08 | 0 | 0 | 0 | 0 | 0 | 0 | 0 | 0 | 0 | 0 | 0 | 0 | 0 |
| 09 | 0 | 0 | 0 | 0 | 0 | 0 | 0 | 0 | 0 | 0 | 0 | 0 | 0 |
| 10 | 0 | 0 | 0 | 0 | 0 | 0 | 0 | 0 | 0 | 0 | 0 | 0 | 0 |
| 11 | 0 | 0 | 0 | 0 | 0 | 0 | 0 | 0 | 0 | 0 | 0 | 0 | 0 |
| 12 | 0 | 0 | 0 | 0 | 0 | 0 | 0 | 0 | 0 | 0 | 0 | 0 | 0 |

* Patch not applied.

EP 0 267 617 B1

Test Substance - VI

| Subject Drug Number | Induction Grades | | | | | | | | | Challenge Grades | | | |
|---|---|---|---|---|---|---|---|---|---|---|---|---|---|
| | 1 | 2 | 3 | 4 | 5 | 6 | 7 | 8 | 9 | Orig 1 | Alt 1 | Orig 2 | Alt 2 |
| 13 | 1 | 0 | 0 | 0 | 0 | 0 | 0 | 0 | 0 | 0 | 1 | 0 | 1 |
| 14 | 0 | 0 | 0 | 0 | 1 | 1 | 1 | 1 | 1 | 1 | 1 | 0 | 0 |
| 15 | 0 | 0 | 0 | 1 | 0 | 0 | 1 | 0 | 0 | 1E | 1E(S) | 1E | 1 |
| 16 | 0 | 0 | 0 | 0 | 1 | 1 | 0 | 0 | 1 | 0 | 0 | 0 | 0 |
| 17 | 0 | 0 | 1 | 0 | 0 | 1 | 0 | 0 | 1 | 0 | 0 | 0 | 0 |
| 18 | 0 | 1 | 1 | 1 | 1 | 1 | 1 | 0 | 1 | 1E | 1E | 1 | 2 |
| 19* | 0 | 0 | ± | ± | ± | - | - | - | - | - | - | - | - |
| 20 | 0 | 0 | 0 | 0 | 0 | 0 | 1 | 1 | 1 | 0 | 1 | 0 | 0 |
| 21 | 0 | 0 | 0 | 0 | 0 | 0 | 0 | 0 | 1 | 1 | 0 | 0 | 0 |
| 22 | 0 | 0 | 0 | 0 | 0 | 0 | 0 | 0 | 0 | 0 | 0 | 0 | 0 |
| 23 | 0 | 0 | 0 | 0 | 0 | 1 | 0 | 0 | 0 | 0 | 0 | 0 | 0 |
| 24 | 0 | 1 | 1E | 2E | 1/1E | 1/2E | 2E/1E | 2/1 | 1/1 | 1E | 1E | 1E | 1E |

* Subject discharged due to viral illness

**Test Substance - VIII**

| Subject Drug Number | Induction Grades | | | | | | | | | Challenge Grades | | | |
|---|---|---|---|---|---|---|---|---|---|---|---|---|---|
| | 1 | 2 | 3 | 4 | 5 | 6 | 7 | 8 | 9 | Orig 1 | Alt 1 | Orig 2 | Alt 2 |
| 13 | 0 | 0 | 0 | 0 | 0 | 0 | 0 | 0 | 0 | 0 | 0 | 0 | 0 |
| 14 | 0 | 0 | 0 | 0 | 0 | 0 | 0 | 0 | 0 | 0 | 0 | 0 | 0 |
| 15 | 0 | 0 | 0 | 0 | 0 | 0 | 0 | 0 | 0 | 0 | 1 | 0 | 0 |
| 16 | 0 | 0 | 0 | 0 | 0 | 0 | 0 | 0 | 0 | 0 | 0 | 0 | 0 |
| 17 | 0 | 0 | 0 | 0 | 0 | 0 | 1 | 0 | 0 | 0 | 0 | 0 | 0 |
| 18 | 0 | 0 | 0 | 0 | 0 | 0 | 0 | 0 | 1 | 0 | 0 | 0 | 0 |
| 19* | 0 | 0 | 0 | 0 | 1 | - | - | - | - | - | - | - | - |
| 20 | 0 | 0 | 0 | 0 | 0 | 1E | 1E | 1 | 0 | 0 | 0 | 0 | 0 |
| 21 | 0 | 0 | 0 | 0 | 0 | 0 | 0 | 0 | 1 | 0 | 0 | 0 | 0 |
| 22 | 0 | 0 | 0 | 0 | 0 | 0 | 0 | 0 | 0 | 1 | 0 | 0 | 0 |
| 23 | 0 | 0 | 0 | 0 | 0 | 0 | 0 | 3 | 0 | 0 | 0 | 0 | 0 |
| 24 | 0 | 0 | 0 | 0 | 0 | 0 | 0 | 0 | 1 | 0 | 0 | 0 | 0 |

\* subject discharged due to viral illness

Because of severe reaction caused by Test Substance #1, no induction patches were applied to Subject No. 5 at the last two induction applications. At challenge, patches free of any test substance were applied to selected sites to evaluate the Subject's reaction to the tape and the possible exacerbation by the action of the test substances.

On Subject No. 6, severe reaction was observed at Test Substance #1 site after the fifth test induction application which spread to all patch sites. After the sixth test induction application, no patches were applied to any test site for the remainder of the induction period. At challenge, patches containing no test substance were applied to selected sites on both arms of the subject.

The testing investigator's conclusions were that, except for Test Substances 1 and 6, the adverse symptomatology was generally mild in nature and no medical intervention was required. All twelve subjects treated with Test substance #1 experienced significant skin changes during the induction period. Nine of the subjects also experienced exacerbation of the skin changes following the challenge patch application. One subject treated with Test Substance #6 experienced significant skin changes both during induction and challenge periods. Two subjects displayed mild reactions to Test Substance #6 during induction which became more severe during challenge. Medical intervention in the form of patch relocation or interruption of patch application was necessary for the significant skin responses to Test Substances #1 and #6.

In conclusion, it is evident that Test Substances #2, #3, and #8, all of which are within the scope of the

EP 0 267 617 B1

present invention, are substantially-free from skin irritation effects. On the other hand, Test Substance #1, from the prior art, and Test Substance #6, which had shown promising penetration enhancement in preliminary testing, both showed significant skin irritation.

Blood samples taken from all twenty-four subjects before and after testing showed no clinically significant changes in serum prolactin levels.

EXAMPLE VII

The following are exemplary of other compositions which can be formulated within the scope of this invention. However, they are illustrative only and are not intended to define the scope of the invention. The compositions can be conventionally formulated simply by mixing all components thoroughly. In some formulations, exact percentages are given whereas others are expressed by ranges. All compositions are in percent by weight.

| FORMULATION VI-A | |
| --- | --- |
| Testosterone | 5-15% |
| Glycerol Dioleate | 50-90% |
| Ethanol | 4-45% |

| FORMULATION VI-B | |
| --- | --- |
| Methadone | 10-30% |
| Glycerol Dioleate | 60-80% |
| Ethanol | 10-30% |

| FORMULATION VI-C | |
| --- | --- |
| Estradiol | 0,1-1.0% |
| Glycerol Dioleate | 60-95% |
| Ethanol | 5-40% |

| FORMULATION VI-D | |
| --- | --- |
| Ketoprofen | 10-20% |
| Glycerol Dioleate | 50-90% |
| Ethanol | 5-40% |

| FORMULATION VI-E | |
| --- | --- |
| Dihydroergotamine | 1-10% |
| Glycerol Dioleate | 50-95% |
| Ethanol | 5-40% |

| FORMULATION VI-F | |
| --- | --- |
| Nifedapine | 2-10% |
| Glycerol Dioleate | 50-95% |
| Ethanol | 5-40% |

18

| FORMULATION VI-G | |
|---|---|
| Thiethylperazine | 1-5% |
| Glycerol Dioleate | 50-95% |
| Ethanol | 5-50% |

| FORMULATION VI-H | |
|---|---|
| Metoclopramide | 10-15% |
| Glycerol Dioleate | 50-90% |
| Ethanol | 5-40% |

| FORMULATION VI-I | |
|---|---|
| Propranolol HCl | 5% |
| Glycerol Dioleate | 75% |
| Ethanol | 20% |

| FORMULATION VI-J | |
|---|---|
| Propranolol | 20% |
| Glycerol Dioleate | 60% |
| Ethanol | 20% |

| FORMULATION VI-K | |
|---|---|
| Propranolol HCl | 5% |
| Glycerol Monooleate | 80% |
| Ethanol | 15% |

| FORMULATION VI-M | |
|---|---|
| Propranolol | 15% |
| Glycerol Trioleate | 65% |
| Isopropanol | 20% |

| FORMULATION VI-N | |
|---|---|
| Fentanyl Citrate | 2% |
| Glycerol Monooleate | 78% |
| Ethanol | 20% |

| FORMULATION VI-O | |
|---|---|
| Fentanyl | 1% |
| Glycerol Trioleate | 79% |
| Isopropanol | 20% |

| FORMULATION VI-Q | |
|---|---|
| Nicardipine HCl | 10% |
| Oleic Acid | 10% |
| Glycerol Dioleate | 50% |
| Ethanol | 30% |

| FORMULATION VI-R | |
|---|---|
| Naloxone HCl | 10% |
| Glycerol Monooleate | 60% |
| Oleic Acid | 10% |
| Ethanol | 20% |

| FORMULATION VI-S | |
|---|---|
| Naloxone | 5% |
| Glycerol Trioleate | 65% |
| Propanol | 30% |

| FORMULATION VI-U | |
|---|---|
| Griseofulvin | 5% |
| Glycerol Trioleate | 65% |
| Isopropanol | 30% |

| FORMULATION VI-W | |
|---|---|
| Fluocinolone Acetonide | 1% |
| Glycerol Trioleate | 69% |
| Isopropanol | 20% |

| FORMULATION VI-Y | |
|---|---|
| Neomycin Sulfate | 5% |
| Glycerol Monooleate | 75% |
| Ethanol | 20% |

| FORMULATION VI-Z | |
|---|---|
| Clonidine HCl | 1% |
| Glycerol Dioleate | 79% |
| Ethanol | 20% |

| FORMULATION VI-AA | |
|---|---|
| Hydroflumethiazide | 10% |
| Glycerol Dioleate | 60% |
| Oleic Acid | 10% |
| Isopropanol | 20% |

| FORMULATION VI-BB | |
|---|---|
| Phentermine | 5% |
| Glycerol Trioleate | 75% |
| Propanol | 20% |

| FORMULATION VI-CC | |
|---|---|
| Phentermine HCl | 10% |
| Glycerol Monooleate | 60% |
| Ethanol | 30% |

| FORMULATION VI-DD | |
|---|---|
| Mazindol | 5% |
| Glycerol Trioleate | 75% |
| Isopropanol | 20% |

| FORMULATION VI-FF | |
|---|---|
| Morphine Sulfate | 5% |
| Glycerol Monooleate | 65% |
| Oleic Acid | 10% |
| Ethanol | 20% |

| FORMULATION VI-GG | |
|---|---|
| Alprazolam | 5% |
| Glycerol Trioleate | 75% |
| Propanol | 20% |

| FORMULATION VI-HH | |
|---|---|
| Ibuprofen | 10% |
| Glycerol Trioleate | 70% |
| Isopropanol | 20% |

| FORMULATION VI-JJ | |
|---|---|
| Naproxen Sodium | 10% |
| Glycerol Monooleate | 70% |
| Ethanol | 20% |

| FORMULATION VI-LL | |
|---|---|
| Methyl Testosterone | 5% |
| Glycerol Trioleate | 65% |
| Isopropanol | 30% |

**Claims**

**Claims for the following Contracting States : AT, BE, CH, DE, FR, GB, IT, LI, LU, NL, SE**

1. A penetration-enhancing pharmaceutical composition for topical application comprising:
   (a) 0.01 to 50% by weight of an active pharmaceutical permeant contained in,
   (b) 40-99.99% by weight of a binary penetration-enhancing vehicle exclusive of diols and N-cyclic solvents comprising
   (i) one or more cell-envelope disordering compounds selected from oleic acid, glycerol monooleate, glycerol dioleate and glycerol trioleate and mixtures thereof, and
   (ii) a lower alkanol selected from ethanol, propanol and isopropanol and mixtures thereof, wherein the weight ratio of cell-envelope disordering compound to lower alkanol is between 50:1 and 1:50; and
   (c) optionally, such inert components as are needed to improve the cosmetic acceptability of the composition and which do not interfere with the penetration enhancement properties of the binary penetration-enhancing vehicle.

2. A composition according to Claim 1 wherein the ratio of cell-envelope disordering compound to lower alkanol is between 9:1 and 1:9.

3. A composition according to Claim 2 wherein active pharamaceutical permeant is present in amounts ranging from 0.01 to 30% by weight and the penetration enhancement vehicle is present in amounts ranging from 70 to 99.99% by weight.

4. A composition according to Claim 3 wherein the active pharmaceutical permeant is a member selected from antimicrobials, antibacterials, antibiotics, antimyobacterials, antimalerials, antimebics, anthelmintics, antifungals, antivirals, neoplastic agents, agents affecting the immune response, blood calcium regulators, peptide and protein hormones, male sex hormones, female sex hormones, agents useful in glucose regulation, anticoagulants, antithrombotics and hemostatics, antihyperlipidemic agents, cardiac drugs, thyromimetic and antithyroid drugs, adrenergics, antihypertensive agents, cholinergics, anticholinergics, antispasmodics, antiulcer agents, skeletal and smooth muscle relaxants, histamine H2-receptor agonists and antagonists, prostaglandins, general inhibitors of the allergic response, antihistamines, local anesthetics, analgesics, antitussives, sedative-hypnotic agents, antidepressant agents, anorexigenics, non-steroidal anti-inflammatory agents, steroidal anti-inflammatory agents, bone-active agents, antiarthritics, vitamins, diagnostic agents, sunscreen agents and compatible mixtures thereof.

5. A composition according to any of Claims 1 to 4 wherein the cell-envelope disordering compound is

22

EP 0 267 617 B1

oleic acid.

6. A composition according to any of Claims 1 to 4 wherein the cell-envelope disordering compound is glycerol monooleate.

7. A composition according to any of Claims 1 to 4 wherein the cell-envelope disordering compound is glycerol dioleate.

8. A composition according to any of Claims 1 to 4 wherein the cell-envelope disordering compound is a mixture of glycerol dioleate and oleic acid.

9. A composition according to any of Claims 1 to 4 wherein the cell-envelope disordering compound is glycerol trioleate.

10. A composition according to any of Claims 1 to 4 wherein the cell-envelope disordering compound is a mixture of glycerol monooleate and oleic acid.

**Claims for the following Contracting States : ES, GR**

1. A method for preparing a penetration-enhancing pharmaceutical composition for topical application comprising:
   (a) 0.01 to 50% by weight of an active pharmaceutical permeant contained in,
   (b) 40-99.99% by weight of a binary penetration-enhancing vehicle exclusive of diols and N-cyclic solvents comprising
      (i) one or more cell-envelope disordering compounds selected from oleic acid, glycerol monooleate, glycerol dioleate and glycerol trioleate and mixtures thereof, and
      (ii) a lower alkanol selected from ethanol, propanol and isopropanol and mixtures thereof, wherein the weight ratio of cell-envelope disordering compound to lower alkanol is between 50:1 and 1:50; and
   (c) optionally, such inert components as are needed to improve the cosmetic acceptability of the composition and which do not interrfere with the penetration enhancement properties of the binary penetration-enhancing vehicle
   by combining the components in a manner known per se.

2. The method according to Claim 1 wherein the ratio of cell-envelope disordering compound to lower alkanol is between 9:1 and 1:9.

3. The method according to Claim 2 wherein active pharamaceutical permeant is present in amounts ranging from 0.01 to 30% by weight and the penetration enhancement vehicle is present in amounts ranging from 70 to 99.99% by weight.

4. The method according to Claim 3 wherein the active pharmaceutical permeant is a member selected from antimicrobials, antibacterials, antibiotics, antimyobacterials, antimalerials, antimebics, anthelmintics, antifungals, antivirals, neoplastic agents, agents affecting the immune response, blood calcium regulators, peptide and protein hormones, male sex hormones, female sex hormones, agents useful in glucose regulation, anticoagulants, antithrombotics and hemostatics, antihyperlipidemic agents, cardiac drugs, thyromimetic and antithyroid drugs, adrenergics, antihypertensive agents, cholinergics, anticholinergics, antispasmodics, antiulcer agents, skeletal and smooth muscle relaxants, histamine H2-receptor agonists and antagonists, prostaglandins, general inhibitors of the allergic response, antihistamines, local anesthetics, analgesics, antitussives, sedative-hypnotic agents, antidepressant agents, anorexigenics, non-steroidal anti-inflammatory agents, steroidal anti-inflammatory agents, bone-active agents, antiarthritics, vitamins, diagnostic agents, sunscreen agents and compatible mixtures thereof.

5. The method according to any of Claims 1 to 4 wherein the cell-envelope disordering compound is oleic acid.

6. The method according to any of Claims 1 to 4 wherein the cell-envelope disordering compound is glycerol monooleate.

**7.** The method according to any of Claims 1 to 4 wherein the cell-envelope disordering compound is glycerol dioleate.

**8.** The method according to any of Claims 1 to 4 wherein the cell-envelope disordering compound is a mixture of glycerol dioleate and oleic acid.

**9.** The method according to any of Claims 1 to 4 wherein the cell-envelope disordering compound is glycerol trioleate.

**10.** The method according to any of Claims 1 to 4 wherein the cell-envelope disordering compound is a mixture of glycerol monooleate and oleic acid.

**Revendications**

**Revendications pour les Etats contractants suivants : AT, BE, CH, DE, FR, GB, IT, LI, LU, NL, SE**

**1.** Composition pharmaceutique renforçant la pénétration pour une application topique comprenant :
(a) 0,01 à 50% en poids d'un agent de perméation pharmaceutique actif contenu dans,
(b) 40 à 99,99% en poids d'un véhicule binaire de renforcement de la pénétration ne contenant ni diols, ni solvants N-cycliques, comprenant :
(i) un ou plusieurs composés modifiant l'enveloppe des cellules choisis parmi l'acide oléique, le monooléate de glycérol, le dioléate de glycérol et le trioléate de glycérol et leurs mélanges, et
(ii) un alcanol inférieur choisi parmi l'éthanol, le propanol, l'isopropanol et leurs mélanges, dans lequel le rapport en poids du composé modifiant l'enveloppe des cellules à l'alcanol inférieur est compris entre 50:1 et 1:50; et
(c) facultativement, les composants inertes qui seraient nécessaires pour améliorer l'acceptabilité cosmétique de la composition et qui n'interfèrent pas avec les propriétés de renforcement de la pénétration du véhicule binaire de renforcement de la pénétration.

**2.** Composition selon la revendication 1, dans laquelle le rapport du composé modifiant l'enveloppe des cellules à l'alcanol inférieur est compris entre 9:1 et 1:9.

**3.** Composition selon la revendication 2, dans laquelle l'agent de perméation pharmaceutique actif est présent en quantités comprises entre 0,01 et 30% en poids et le véhicule de renforcement de la pénétration est présent en quantités comprises entre 70 et 99,99% en poids.

**4.** Composition selon la revendication 3, dans laquelle l'agent de perméation pharmaceutiquement actif est un élément choisi parmi les suivants : antimicrobiens, antibactériens, antibiotiques, antimyobactériens, antipaludéens, antiamibiens, vermifuges, antifongiques, antiviraux, agents néoplastiques, agents affectant la réponse immunitaire, régulateurs du calcium sanguin, hormones à base de peptides et de protéines, hormones mâles, hormones femelles, agents utiles dans la régulation du glucose, anticoagulants, antithrombotiques et hémostatiques, agents hypolipidémiques, médicaments pour le coeur, médicaments thyromimétiques et antithyroïdiens, adrénergiques, agents hypotenseurs, cholinergiques, anticholinergiques, antispasmodiques, agents anti-ulcères, décontractants des muscles du squelette et des muscles lisses, histamine, agonistes et antagonistes récepteurs de H2, prostaglandines, inhibiteurs généraux de la réponse allergique, anti-histaminiques,anesthésiques locaux, analgésiques, antitussifs, agents sédatifs-hypnotiques, agents antidépresseurs, anorexigènes, agents anti-inflammatoires non-stéroïdaux, agents anti-inflammatoires stéroïdaux, agents actifs sur les os,anti-arthritiques, vitamines, agents diagnostiques, écrans solaires et leurs mélanges compatibles.

**5.** Composition selon l'une des revendications 1 à 4, dans laquelle le composé modifiant l'enveloppe des cellules est l'acide oléique.

**6.** Composition selon l'une des revendications 1 à 4, dans laquelle l'agent modifiant l'enveloppe des cellules est le monooléate de glycérol.

**7.** Composition selon l'une des revendications 1 à 4, dans laquelle le composé modifiant l'enveloppe des cellules est le dioléate de glycérol.

**8.** Composition selon l'une des revendications 1 à 4, dans laquelle le composé modifiant l'enveloppe des cellules est un mélange de dioléate de glycérol et d'acide oléique.

**9.** Composition selon l'une des revendications 1 à 4, dans laquelle le composé modifiant l'enveloppe des cellules est le trioléate de glycérol.

**10.** Composition selon l'une des revendications 1 à 4, dans laquelle le composé modifiant l'enveloppe des cellules est un mélange de monooléate de glycérol et d'acide oléique.

**Revendications pour les Etats contractants suivants : ES, GR**

**1.** Méthode pour préparer une composition pharmaceutique de renforcement de la pénétration pour application topique, comprenant :

(a) 0,01 à 50% en poids d'un agent de perméation pharmaceutique actif contenu dans :

(b) 40 à 99,99% en poids d'un véhicule binaire de renforcement de la pénétration, à l'exclusion de diols et de solvants N-cycliques, comprenant :

(i) un ou plusieurs composés modifiant l'enveloppe des cellules choisis parmi l'acide oléique, le monooléate de glycérol, le dioléate de glycérol, le trioléate de glycérol et leurs mélanges, et

(ii) un alcanol inférieur choisi parmi l'éthanol, le propanol, l'isopropanol et leurs mélanges, dans lequel le rapport en poids du composé modifiant l'enveloppe des cellules à l'alcanol inférieur est compris entre 50:1 et 1:50; et

(c) facultativement, les composants inertes qui seraient nécessaires pour améliorer l'acceptabilité cosmétique de la composition et qui n'interfèrent pas avec les propriétés de renforcement de la pénétration du véhicule binaire de renforcement de la pénétration,

en combinant les composants d'une manière connue en soi.

**2.** Méthode selon la revendication 1, dans laquelle le rapport du composé modifiant l'enveloppe des cellules à l'alcanol inférieur est compris entre 9:1 et 1:9.

**3.** Méthode selon la revendication 2, dans laquelle l'agent de perméation pharmaceutique actif est présent en quantités comprises entre 0,01 et 30% en poids et le véhicule de renforcement de la pénétration est présent en quantités comprises entre 70 et 99,99% en poids.

**4.** Méthode selon la revendication 3, dans laquelle l'agent de perméation pharmaceutique actif est un élément choisi parmi les suivants : antimicrobiens, antibactériens, antibiotiques, antimyobactériens, antipaludéens, antiamibiens, vermifuges, antifongiques, antiviraux, agents néoplastiques, agents affectant la réponse immunitaire, régulateurs du calcium sanguin, hormones à base de peptides et de protéines, hormones mâles, hormones femelles, agents utiles dans la régulation du glucose, anticoagulants, antithrombotiques et hémostatiques, agents hypolipidémiques, médicaments pour le coeur, médicaments thyromimétiques et antithyroïdiens, adrénergiques, agents hypotenseurs, cholinergiques, anticholinergiques, antispasmodiques, agents anti-ulcères, décontractants des muscles du squelette et des muscles lisses, histamine, agonistes et antagonistes récepteurs de H2, prostaglandines, inhibiteurs généraux de la réponse allergique, antihistaminiques, anesthésiques locaux, analgésiques, antitussifs, agents sédatifs-hypnotiques, agents antidépresseurs, anorexigènes, agents anti-inflammatoires non stéroïdaux, agents anti-inflammatoires stéroïdaux, agents actifs sur les os, anti-arthritiques, vitamines, agents diagnostiques, écrans solaires et leurs mélanges compatibles.

**5.** Méthode selon l'une des revendications 1 à 4, dans laquelle le composé modifiant l'enveloppe des cellules est l'acide oléique.

**6.** Méthode selon l'une des revendications 1 à 4, dans laquelle le composé modifiant l'enveloppe des cellules est le monooléate de glycérol.

**7.** Méthode selon l'une des revendications 1 à 4, dans laquelle le composé modifiant l'enveloppe des cellules est le dioléate de glycérol.

**8.** Méthode selon l'une des revendications 1 à 4, dans laquelle le composé modifiant l'enveloppe des cellules est un mélange de dioléate de glycérol et d'acide oléique.

9. Méthode selon l'une des revendications 1 à 4, dans laquelle le composé modifiant l'enveloppe des cellules est le trioléate de glycérol.

10. Méthode selon l'une des revendications 1 à 4, dans laquelle le composé modifiant l'enveloppe des cellules est un mélange de monooléate de glycérol et d'acide oléique.

**Patentansprüche**
**Patentansprüche für folgende Vertragsstaaten : AT, BE, CH, DE, FR, GB, IT, LI, LU, NL, SE**

1. Eine die Penetration verstärkende pharmazeutische Zusammensetzung zur lokalen Anwendung, umfassend:
    (a) 0,01 bis 50 Gew.-% eines durchdringenden Wirkstoffs, enthalten in
    (b) 40-99,99 Gew.-% eines binären, die Penetration verstärkenden Vehikels ohne Diole und N-zyklische Lösungsmittel, umfassend
       (i) eine oder mehrere die Zellhülle störende Verbindungen, ausgewählt aus Ölsäure, Glycerinmonooleat, Glycerimdioleat und Glyerintrioleat und Mischungen davon, und
       (ii) einen niederen Alkanol ausgewählt aus Ethanol, Propanol und Isopropanol und Mischungen davon, worin das Gewichtsverhältnis von die Zellhülle störender Verbindung zu niederem Alkanol zwischen 50:1 und 1:50 liegt; und
    (c) gegebenenfalls solche inerten Bestandteile, wie sie benötigt werden, um die kosmetische Verträglichkeit der Zusammensetzung zu erhöhen, und die die penetrationverstärkenden Eigenschaften des binären, die Penetration verstärkenden Vehikels nicht beeinflussen.

2. Eine Zusammensetzung gemäß Anspruch 1, worin das Verhältnis der die Zellhülle störenden Verbindung zu niederem Alkanol zwischen 9:1 und 1:9 liegt.

3. Eine Zusammensetzung gemäß Anspruch 2, worin der durchdringende Wirkstoff in Mengen zwischen 0,01 bis 30 Gew.-% vorhanden ist, und das die Penetration verstärkende Vehikel ist in Mengen zwischen 70 bis 99,99 Gew.-% vorhanden.

4. Eine Zusammensetzung gemäß Anspruch 3, worin der durchdringende Wirkstoff ein Mitglied ist, ausgewählt aus antimikrobielle Mittel, Antibiotika, antimyobakterielle Mittel, Antimalariamittel, antimebische Stoffe, Anthelmintika, Antipilzmittel, antivirale Mittel, neoplastische Mittel, Mittel, die die Immunantwort beeinflussen, Blutkalziumregulatoren, Peptid- und Proteinhormone, männliche Sexualhormone, weibliche Sexualhormone, Mittel, die zur Glucoseregulation nützlich sind, Antikoagulanzien, Antithrommotika und Hämostatika, Antihyperlipidmittel, Herzmittel, Thyromimetika und Antithyroidmittel, Adrenergetika, antihypertensive Mittel, Cholinergika, Anticholinergika, krampflösende Mittel, Mittel gegen Geschwüre, Relaxanzien für die Skelett- und glatte Muskulatur, Agonisten und Antagonisten für Histamin-H2-Rezeptor, Prostaglandine, allgemeine Inhibitoren der allergischen Reaktion, Antihistaminika, lokale Anästhetika, Analgetika, Mittel gegen Hustenreiz, sedativ-hypnotische Mittel, Antidepressiva, Mittel gegen Anorexie, nicht-steroidartige entzündungshemmende Mittel, steroidartige entzündungshemmende Mittel, auf die Knochen wirkende Mittel, Antiarthritika, Vitamine, Diagnosemittel, Sonnenschutzmittel und verträgliche Mischungen davon.

5. Eine Zusammensetzung gemäß einem der Ansprüche 1 bis 4, worin die die Zellhülle störende Verbindung Ölsäure ist.

6. Eine Zusammensetzung gemäß einem der Ansprüche 1 bis 4, worin die die Zellhülle störende Verbindung Glycerinmonooleat ist.

7. Eine Zusammensetzung gemäß einem der Ansprüche 1 bis 4, worin die die Zellhülle störende Verbindung Glycerindioleat ist.

8. Eine Zusammensetzung gemäß einem der Ansprüche 1 bis 4, worin die die Zellhülle störende Verbindung eine Mischung aus Glycerindioleat und Ölsäure ist.

9. Eine Zusammensetzung gemäß einem der Ansprüche 1 bis 4, worin die die Zellhülle störende Verbindung Glycerintrioleat ist.

**10.** Eine Zusammensetzung gemäß einem der Ansprüche 1 bis 4, worin die die Zellhülle störende Verbindung eine Mischung aus Glycerinmonooleat und Ölsäure ist.

**Patentansprüche für folgende Vertragsstaaten : ES, GR**

**1.** Ein Verfahren zum Herstellen einer die Penetration verstärkenden Zusammensetzung zur lokalen Anwendung, umfassend:

(a) 0,01 bis 50 Gew.-% eines durchdringenden Wirkstoffs, enthalten in

(b) 40-99,99 Gew.-% eines binären, die Penetration verstärkenden Vehikels ohne Diole und N-zyklische Lösungsmittel, umfassend

(i) eine oder mehrere die Zellhülle störende Verbindungen, ausgewählt aus Ölsäure, Glycerinmonooleat, Glycerimdioleat und Glyerintrioleat und Mischungen davon, und

(ii) einen niederen Alkanol ausgewählt aus Ethanol, Propanol und Isopropanol und Mischungen davon, worin das Gewichtsverhältnis von die Zellhülle störender Verbindung zu niederem Alkanol zwischen 50:1 und 1:50 liegt; und

(c) gegebenenfalls solche inerten Bestandteile, wie sie benötigt werden, um die kosmetische Verträglichkeit der Zusammensetzung zu erhöhen, und die die penetrationverstärkenden Eigenschaften des binären, die Penetration verstärkenden Vehikels nicht beeinflussen,

durch Kombinieren der Bestandteile in bekannter Weise.

**2.** Das Verfahren nach Anspruch 1, worin das Verhältnis der die Zellhülle störenden Verbindung zu niederem Alkanol zwischen 9:1 und 1:9 liegt.

**3.** Das Verfahren gemäß Anspruch 2, worin der durchdringende Wirkstoff in Mengen zwischen 0,01 bis 30 Gew.-% vorhanden ist, und das die Penetration verstärkende Vehikel ist in Mengen zwischen 70 bis 99,99 Gew.-% vorhanden.

**4.** Das Verfahren gemäß Anspruch 3, worin der durchdringende Wirkstoff ein Mitglied ist, ausgewählt aus antimikrobielle Mittel, Antibiotika, antimyobakterielle Mittel, Antimalariamittel, antimebische Mittel, Anthelmintika, Antipilzmittel, antivirale Mittel, neoplastische Mittel, Mittel, die die Immunantwort beeinflussen, Blutkalziumregulatoren, Peptid- und Proteinhormone, männliche Sexualhormone, weibliche Sexualhormone, Mittel, die zur Glucoseregulation nützlich sind, Antikoagulanzien Antithrombotika und Hämostatika, Antihyperlipidmittel, Herzmittel, Thyromimetika und Antithyroidmittel, Adrenergetika, antihypertensive Mittel, Cholinergika, Anticholinergika, krampflösende Mittel, Mittel gegen Geschwüre, Relaxanzien für die Skelett- und glatte Muskulatur, Agonisten und Antagonisten für Histamin-H2-Rezeptor, Prostaglandine, allgemeine Inhibitoren der allergischen Reaktion, Antihistaminika, lokale Anästhetika, Analgetika, Mittel gegen Hustenreiz, sedativ-hypnotische Mittel, Antidepressiva, Mittel gegen Anorexie, nicht-steroidartige entzündungshemmende Mittel, steroidartige entzündungshemmende Mittel, auf die Knochen wirkende Mittel, Antiarthritika, Vitamine, Diagnosemittel, Sonnenschutzmittel und verträgliche Mischungen davon.

**5.** Das Verfahren gemäß einem der Ansprüche 1 bis 4, worin die die Zellhülle störende Verbindung Ölsäure ist.

**6.** Das Verfahren nach einem der Ansprüche 1 bis 4, worin die die Zellhülle störende Verbindung Glycerinmonooleat ist.

**7.** Das Verfahren nach einem der Ansprüche 1 bis 4, worin die die Zellhülle störende Verbindung Glycerindioleat ist.

**8.** Das Verfahren gemäß einem der Ansprüche 1 bis 4, worin die die Zellhülle störende Verbindung eine Mischung aus Glycerindioleat und Ölsäure ist.

**9.** Das Verfahren gemäß einem der Ansprüche 1 bis 4, worin die die Zellhülle störende Verbindung Glycerintrioleat ist.

**10.** Das Verfahren gemäß einem der Ansprüche 1 bis 4, worin die die Zellhülle störende Verbindung eine Mischung aus Glycerinmonooleat und Ölsäure ist.